(19) **Europäisches Patentamt / European Patent Office / Office européen des brevets**

(11) **EP 1 848 997 B1**

(12) **EUROPEAN PATENT SPECIFICATION**

(45) Date of publication and mention of the grant of the patent:
**21.11.2012 Bulletin 2012/47**

(51) Int Cl.:
**G01N 33/53** (2006.01)

(21) Application number: **06715833.7**

(22) Date of filing: **03.02.2006**

(86) International application number:
**PCT/KR2006/000380**

(87) International publication number:
**WO 2006/083126 (10.08.2006 Gazette 2006/32)**

(54) **COMPOSITION FOR PREVENTION, TREATMENT, AND DIAGNOSIS OF CHRONIC OBSTRUCTIVE PULMONARY DISEASE (COPD)**

ZUSAMMENSETZUNG ZUR VORBEUGUNG, BEHANDLUNG UND DIAGNOSE VON COPD (CHRONIC OBSTRUCTIVE PULMONARY DISEASE)

COMPOSITION CONVENANT POUR LA PREVENTION, LE TRAITEMENT ET LE DIAGNOSTIC DE LA BRONCHO-PNEUMOPATHIE CHRONIQUE OBSTRUCTIVE (BPCO)

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.02.2005 KR 20050010924**
**02.02.2006 KR 20060010285**

(43) Date of publication of application:
**31.10.2007 Bulletin 2007/44**

(73) Proprietors:
• **Jeon, Sook-yeong**
**Yongin-si,**
**Gyeonggi-do 449-843 (KR)**
• **Nahm, Dong-ho**
**Yongin-si,**
**Gyeonggi-do 449-843 (KR)**

(72) Inventors:
• **Jeon, Sook-yeong**
**Yongin-si,**
**Gyeonggi-do 449-843 (KR)**
• **Nahm, Dong-ho**
**Yongin-si,**
**Gyeonggi-do 449-843 (KR)**

(74) Representative: **Rystedt, Per Hampus**
**BRANN AB**
**P.O. Box 12246**
**102 26 Stockholm (SE)**

(56) References cited:

WO-A-03/023002    WO-A-03/098211
WO-A-2006/073254

• RYU J H ET AL: "Obstructive lung diseases: COPD, asthma, and many imitators." MAYO CLINIC PROCEEDINGS. MAYO CLINIC NOV 2001, vol. 76, no. 11, November 2001 (2001-11), pages 1144-1153, XP002491921 ISSN: 0025-6196
• JEFFERY PETER K: "Remodeling and inflammation of bronchi in asthma and chronic obstructive pulmonary disease." 2004, PROCEEDINGS OF THE AMERICAN THORACIC SOCIETY 2004, VOL. 1, NR. 3, PAGE(S) 176 - 183 , XP002491922 ISSN: 1546-3222 * the whole document *
• DATABASE GENBANK [Online] October 2004 KALNINE N. ET AL., XP003018760 Database accession no. (AAV38219)
• NAHM D.-H. ET AL.: 'Identification of Cytokeratin 18 as a Bronchial Epithelial Autoantigen Associated with Nonallergic Asthma' AMERICAN J. RESPIRATORY CRITICAL CARE MEDICINE vol. 165, no. 11, 2002, pages 1536 - 1539, XP002904967
• KANAZAWA H. ET AL.: 'CYFRA 21-1, a Cytokeratin Subunit 19 Fragment, in Bronchoalveolar Lavage Fluid from Patients with Interstitial Lung Disease' CLINICAL SCIENCE vol. 94, 1998, pages 531 - 535, XP002217297
• VEERAMACHANENI R. ET AL.: 'Benign Myoepithelioma of the Lung' ARCHIVES PATHOLOGY LABORATORY MEDICINE vol. 125, November 2001, pages 1494 - 1496, XP003018761

**Description**

Background of the Invention

Technical Field

The present invention relates to compositions for prevention, treatment, and diagnosis of chronic obstructive pulmonary disease (COPD).

Background Art

Chronic obstructive pulmonary disease (COPD) is currently defined as chronic disease showing irreversible obstruction of airways resulting from the progression of two major underlying diseases including chronic bronchitis and pulmonary emphysema. Chronic bronchitis is defined clinically as the persistence of cough, sputum, and difficult breathing, an pulmonary emphysema is defined histopathologically as an irrevesible destruction of airway walls distal to the terminal bronchiole and clinically shows slowly progressive respiratory difficulties (GOLD workshop summary, Am J Respir Crit Care Med 2001; 163:1256-1276). Therefore, current definition of COPD excludes a bronchial asthma showing reversible obstruction of airways. COPD is currently the fourth leading cause of death in the United States and Europe, and causes of death in patients with COPD are complications of the disease such as respiratory failure or infection (GOLD workshop summary, Am J Respir Crit Care Med 2001; 163:1256-1276).

Currently, smoking is regarded as the most important risk factor for the development of COPD. And secretions of many inflammatory mediators (IL-8 etc.) from airway epithelial cells stimulated by smoking and other factors including air pollution or chronic infection by bacteria or virus are considered to be responsible for the development of chronic inflammation of airway tissue in COPD (GOLD workshop summary, Am J Respir Crit Care Med 2001; 163:1256-1276). However, the reason for the chronic persistence of inflammation after acute inflammation induced by those external stimuli is not determined yet. Accordingly, it is suggested that other pathogenetic mechanisms besides smoking may be involved in the pathogenetic mechanism of COPD (0'Byrne PM, Postma DS. Am J Respir Crit Care Med 1999; 159: S41-S66). Especially, the reasons for the persistence of airway inflammation and apoptosis of airway epithelial cells in COPD patients even after stop of smoking are not explained yet (Hodge S et al., Eur Respir J 2005;25:447-54). The etiology and pathogenetic mechanism of COPD are not determined yet, and therefore, fundamental treatment of COPD is difficult now. The COPD patients having advanced disease and severely decreased pulmonary function are at a high risk of the disease-associated premature death (GOLD workshop summary, Am J Respir Crit Care Med 2001;163: 1256-1276).

Currently, diagnosis of chronic obstructive pulmonary disease can be made when the patients complain of typical clinical symptoms (cough, sputum, and dyspnea, etc.) and their FEV1 measured following the inhalation of bronchodilator showed less than 80% of predictive value and the ratio of FEV1/FVC was less than 70% on pulmonary function test (GOLD workshop summary, Am J Respir Crit Care Med 2001;163:1256-1276). However, there is still no laboratory test which can be used for the early detection of patients with COPD who complain of typical clinical symptoms but show normal pulmonary function and therby used for the prevention of further progession of COPD.

As a pharmacological therapy of COPD, bronchodilator and corticosteroid are known to be effective for the improvement of clinical symptoms. Direct administration of corticosteroid to the target airway tissue by inhalation devices is preferred method over systemic administration to avoid systemic side effects. Additional medications such as theophylline can also be useful. However, there is still no available therapeutic agent which can induce complete remission of COPD or fundamentally improve COPD and modify a natural course of the disease.

Detection of autoantibodies to antigens of airway or lung tissues in COPD patients has been reported (Wagner V, et al., Acta Allergol 1965;20:1-9). Although a hypothesis that COPD is caused by autoimmune mechanism has been proposed (Agusti A, et al., Thorax 2003;58:832-834), this hypothesis has not been clearly demonstrated because an autoantigen reacting with autoantibodies in the blood of patients with COPD has not been identified yet. Recently, it has been demonstrated that pulmonary emphysema (one of major underlying diseases causing COPD) could have been developed in animals by autoimmune response against the vascular endothelial autoantigen (Taraseviciene-Stewart L, et al., Am J Respir Crit Care Med. 2005;171:734-42). In the above study, it has been reported that rats immunized by antigens extracted from human vascular endothelial cells to induce autoimmune response developed pulmonary emphysema, and passive transfer of CD4+ cells from spleens of the above pulmonary emphysema-developed rats to naive rats resulted in pulmonary emphysema in recipient rats, and pulmonary emphysema has been also caused in mouse after injection of anti-endothelial cell antibodies obtained from serum of rats immunized with vascular endothelial cells (Taraseviciene-Stewart L, et al., Am J Respir Crit Care Med. 2005;171:734-42). This study showed that antibodies to vascular endothelial cell antigens were sufficient to trigger the development of pulmonary emphysema, although target autoantigens involved in development of the disease was not identified yet. On the basis of aboves, it has been suggested that autoimmune mechanism might be involved in the pathogenesis of COPD.

Disclosure

Technical problem

However, previous studies could not establish a causal relationship between autoimmunity and COPD due to the lack of an identified autoantigen associated with patients with COPD and lack of a logical association between the autoantibodies in the bodily fluid of patients with COPD and the damage of airway tissue. So, autoantibody testing is not currently used for the diagnosis or classification of COPD

Because the primary etiology and mechanism causing the development of COPD is not completely understood yet, a treatment method that can induce complete remission of COPD has not been developed yet. Current pharmacological therapy for COPD can improve the clinical symptoms and pulmonary function only during the continuous administration of medication, but there is no treatment method which can modify the long-term natural course of COPD or prevent a death due to COPD.

A cytokeratin 18 protein, which is identified as a target autoantigen of COPD, is a cytoskeletal protein found primarily in epithelial cells lining the respiratory and gastrointestinal tracts, including bronchial epithelial cells and lung (alveolar) epithelial cells (Moll, R. et al., Cell 1982; 31:11-24). Although cytokeratin 18 is a predominantly intracellular protein, its strong expression on the cell surface was also observed in epithelial cells (Moll, R. et al., Cell 1982; 31:11-24; Saarloos, M.N. et al., Curr Eye Res 1999; 19:439-449).

Technical Solution

The present inventors judged that autoantigen proteins involved in the development of COPD might be present in the airway epithelial cells on the basis of previous reports and results from deductive ratiocination of present inventors, and therefore, analyzed the airway epithelial autoantigen proteins reacting with IgG autoantibodies by Immunoblot method using proteins from cultured human airway epithelial cells.

Consequently, the inventors discovered autoantibodies to airway epithelial cells in serum samples of patients with COPD, and identified that the airway epithelial autoantigen was cytokeratin 18 protein. Also, inventors demonstrated significant inhibitions of autoantibody-induced cytotoxicity of airway epithelial cells by adsorption of autoantibodies

WO 03/098211 concerns the detection of autoantibodies to cytokeratin 18 protein in patients with bronchial asthma and chronic rhinitis, and its applications including a kit for diagnosing bronchial asthma and chronic rhinitis comprising mammalian cytokeratin 18 protein. WO 2006/073254 concerns composition for prevention, treatment and diagnosis of chronic inflammatory airway diseases and is an Article 54(3) EPC document in regard to the current invention. WO 03/023002 concerns novel human proteins, polynucleotides encoding them and methods using the same. Ryu and Scanlon (i.e. Mayo Clin. Proc. 2001: 76: 1144-1153) concerns obstructive lung diseases: chronic obstructive pulmonary disease, asthma, and many imitators. Jeffery (i.e. Proc Am Thorac Soc vol 1. pp 176-183, 2004) concerns remodelling and inflammation of bronchi in asthma and chronic obstructive pulmonary disease. from patients with COPD with cytokeratin 18 protein, and thereby the inventors made the present invention.

It is an object of the present invention to provide a pharmaceutical composition for preventing or treating COPD comprising cytokeratin 18 protein as an active ingredient, wherein the cytokeratin 18 protein has an amino acid sequence of SEQ ID NO: 2.

The present invention provides a pharmaceutical composition for preventing or treating COPD comprising cytokeratin 18 protein as an active ingredient, wherein the cytokeratin 18 protein has an amino acid sequence of SEQ ID NO: 2.

Cytokeratin 18 protein used in the present invention has an amino acid sequence of SEQ ID NO: 2.

The amino acid sequence of SEQ ID NO: 1 is an amino acid sequence of human (*Homo sapiens*) cytokeratin 18 protein (NCBI accession no. P05783), and an amino acid sequence of SEQ ID NO: 2 is an amino acid sequence of bovine (*Bos taurus*) cytokeratin 18 protein (NCBI accession no. XP_582930).

Cytokeratin 18 protein can be produced by recombinant genetic engineering technology using one of DNA base sequence encoding the amino acid sequence of SEQ ID NO: 2 , therby isolating them from cells or tissues of mammals, or microorganisms and purifying them.

The "Chronic obstructive pulmonary disease (COPD)" in the present invention includes pulmonary emphysema and chronic bronchitis.

The present invention is based on the fact identifying cytokeratin 18 protein as an airway epithelial autoantigen binding with IgG autoantibodies in the blood samples of patients with COPD, for the first time.

It can be judged from the previous reports and results of Examples in the present invention that autoantibodies in the blood of patients with COPD can directly induce cytotoxicity to airway epithelial cells through binding to cytokeratin 18 proteins of airway epithelial cells or indirectly induce cytotoxicity to airway epithelial cells and induce chronic inflammation of airway tissue through the formation of immune complexes consisted of autoantibodies and autoantigen and secondary complement activation and chemotaxis of inflammatory cells. And these damages of airway epithelial cells and chronic inflammation of airway tissue can cause clinical symptoms of COPD through the developments of irreversible structural changes of airway tissues.

In the Examples of the present invention, IgG antibodies purified from blood samples of patients with COPD induced cytotoxicity to airway epithelial cells. Also, the administration of cytokeratin 18 protein of the present invention into the model of IgG antibody-induced airway epithelial cell cytotoxicity, induced adsorptions of autoantibodies in the blood of patients with COPD, and thereby inhibited IgG antibody-induced cytotoxicity to airway epithelial cells.

Thus, the pharmaceutical compositon comprising cytokeratin 18 protein wherein the cytokeratin 18 protein has an amino acid sequence of SEQ ID NO: 2 of the present invention can be used as a medicament preventing, alleviating or treating COPD.

The pharmaceutical compositon comprising cytokeratin 18 protein wherein the cytokeratin 18 protein has an amino acid sequence of SEQ ID NO: 2 as an active ingredient may further comprise pharmaceutically and physiologically acceptable additives besides the active ingredient. Such additives may include, for example, excipients, disintergrating agents, sweeting agents, binding agents, coating agents, inflating agents, lubricants, glidants, flavoring agents, solubilizers, etc.

The pharmaceutical composition comprising cytokeratin 18 protein wherein the cytokeratin 18 protein has an amino acid sequence of SEQ ID NO: 2 as an active ingredient may further comprise one or more pharmaceutically acceptable carriers to be formulated appropriately for administration.

For liquid formulation, the pharmaceutically acceptable carriers should be sterilized and suitable to living bodies. For example, the pharmaceutically acceptable carriers may include saline, sterilized water, linger solution, bufferd saline, albumin injection solution, dextrose solution, malto dextrine solution, glycerin, ethanol, or the mixture of one or more of the above ingredients. If necessary, other common additives can be added, such as antioxidants, buffers, bacteriostatic agents, etc. Also, diluting agents, dispersing agents, surfactants, binders or lubricants can be further added in order to formulate the composition to injection formulations such as aquous solution, suspension, emulsion, pills, capsules, granules, Tablets, etc. Further more, the present pharmaceutical compositon can be appropriately formulated depending on each disease or ingredient of the composition, by using the disclosed method in Remington's Pharmaceutical Science, Mack Publishing Company, Easton PA, as a preferable method in the art.

Formulation type of the present pharmaceutical compositon comprising cytokeratin 18 protein as an active ingredient, can be granules, powder, coated tablets, tablets, capsules, suppositories, syrup, juice, suspensions, emulsions, drops, injectable liquid formulation or slowly-released formulation of active compound, etc.

The present pharmaceutical compositon comprising cytokeratin 18 protein as an active ingredient, can be administered intravenously, intraarterially, intraperitoneally, intramuscularly, intrasternally, percutaneously, intranasally, rectally, orally, intraocularly, intradermally, locally, or by inhalation, according to ordinary methods.

The dosage of the present pharmaceutical composition comprising cytokeratin 18 protein wherein the cytokeratin 18 protein has an amino acid sequence of SEQ ID NO: 2 as an active ingredient means an effectective amount to inhibit or treat damage or inflammatory reaction of airway epithelial cells and tissues. Thus, this dosage can be modified depending on various factors such as the kind of a disease to be treated, severity of the disease, kinds and amounts of active ingredients and other ingredients contained in the composition, age, weight, general health status and sex of the patient, diet, time and route of administration, secretion rate of the composition, period of treatment drugs used simultaneously, etc. In adults, when cytokeratin 18 protein wherein the cytokeratin 18 protein has an amino acid sequence of SEQ ID NO: 2 is administrated one or several times per day, the preferable dosage of said cytokeratin 18 protein is 0.01 mg/kg-100 mg/kg.

Also, the present invention provides a method for preventing or treating COPD by administering cytokeratin 18 protein wherein the cytokeratin 18 protein has an amino acid sequence of SEQ ID NO: 2.. Preferably, the above COPD can be pulmonary emphysema or chronic bronchitis.

The present specification shows that the administration of cytokeratin 18 proteins can adsorb autoantibodies in the blood of patients with COPD thereby inhibiting autoantibody-induced cytotoxicity to airway epithelial cells and inhibiting the development of secondary inflammation of airway tissue.

The present invention provides a method of preventing or treating COPD by administering cytokeratin 18 protein. In this method, cytokeratin 18 protein wherein the cytokeratin 18 protein has an amino acid sequence of SEQ ID NO: 2 can be administrated intravenously, intraarterially, intraperitoneally, intramuscular, intrasternally, percutaneously, intranasally, rectally, orally, intraocularly, intradermally, locally, or by inhalation, according to ordinary methods.

In the method of preventing or treating COPD by administering cytokeratin 18 protein wherein the cytokeratin 18 protein has an amino acid sequence of SEQ ID NO: 2, the dosage of said cytokeratin 18 protein to be administrated can be referred as an effectective amount to inhibit or treat autoantibody-induced cytotoxicity to airway epithelial cells or secondary inflammatory reaction of airway tissues caused by the formation of immune complex consisted of autoantigen-autoantibodies. In adults, when said cytokeratin 18 protein is administrated one or several times per day, the preferable dosage of cytokeratin 18 proteins is 0.01 mg/kg - 100 mg/kg. This dosage can be modified depending on various factors such as the kind of a disease to be treated, severity of the disease, kinds and amounts of active ingredients and other ingredients contained in the composition, age, weight, general health status and sex of the patient, diet, time or route of administration, secretion rate of composition, period of treatment, drugs used simultaneously, etc.

Also, the present invention provides the use of cytokeratin 18 protein wherein the cytokeratin 18 protein has an amino acid sequence of SEQ ID NO: 2. for the manufacture of a medicament for COPD.

The diagnostic composition comprising cytokeratin 18 protein as disclosed herein can react with biological samples of patients with COPD, thereby showing positive results. Thus, the diagnostic composition can be used for the diagnosis of COPD.

The diagnostic composition disclosed herein comprising cytokeratin 18 protein wherein the cytokeratin 18 protein has an amino acid sequence of SEQ ID NO: 2 can be used for the detection, diagnosis, and classification of COPD with higher sensitivity as shown in the present Examples.

The diagnostic composition can comprise buffer or reaction solution which makes maintain physiological acivity or structure of proteins, besides the proteins. Also, the diagnostic composition can be provided in the form of powder, or in the solubilized state in an appropriate buffer, or maintained at 4°C, in order to maintain stability.

A diagnostic kit for diagnosing COPD is described comprising diagnostic composition comprising cytokeratin 18 protein for diagnosing COPD.

The diagnostic kit can comprise buffer or reaction solution which makes to maintain physiological acivity or structure of proteins, besides cytokeratin 18 proteins. Further, the proteins can be provided in the form of powder, or in the solubilized state in an appropriate buffer, or maintained at 4°C, in order to maintain stability.

The diagnostic kit can comprise components other than cytokeratin 18 protein. For the positive control result, the diagnostic kit can comprise polyclonal antibodies or monoclonal antibodies to cytokeratin 18 protein from mammals, or human's biological samples of which autoantibodies to cytokeratin 18 protein are positive. For the negative control result, the diagnostic kit can comprise other antibodies or buffers. If necessary, the diagnostic kit can further comprise other components required for the detection of autoantibodies to cytokeratin 18 protein from biological samples of subjects.

Other components required for immunodetecting reaction include anti-human IgG antibodies produced from rat, mouse, rabbit, cow, pig, or goat, etc., as the secondary antibodies, and colorizing agents, buffers, etc.

As the secondary antibodies, alkaline phosphatase (AP)-conjugated, horse radish peroxidase (HRP)-conjugated, biotin-conjugated antibodies, or fluorescent material(for example, rhodamine, Texas Red, fluorescein, phycoerythrin, etc.)-conjugated antibodies can be used.

The biotin-conjugated secondary antibodies can use AP or HRF enzyme-conjugated avidin, AP-conjugated secondary antibodies can use BCIP/NBT as color couplers, and HPR-conjugated secondary antibodies can use DAB (diaminobenzidine), etc. as color couplers, thereby inducing color reaction to determine the presence of autoantibodies. The secondary antibodies to which fluorescent materials are conjugated, can be monitored on a fluorescence microscope. Thus, from the fluorescence microscopy, the presence of autoantibodies can be determined.

The assay methods applied to the kit are known to those skilled in the art. The assay methods to be used for the kit include any techniques that can detect antigen-antibody reaction, such as fluorescence immunoassays, enzyme-substrate color reaction, eyzyme immunoassays, immunoblotting, immunoprecipitation assay, antigen-antibody agglutination immunoassays, light-scattering immunoassays, radioimmunoassay, flow cytometry, complement-fixing method, etc.

The kit can further comprise tubes, wells or plates required for mixing each component, or covering letter describing the way of using, if necessary.

The present invention provides an *in vitro* method of diagnosing chronic obstructive pulmonary disease by using diagnostic compositions containing cytokeratin 18 protein as an active ingredient, comprising the steps of:

(a) contacting a biological sample of a subject suspected to have chronic obstructive pulmonary disease, such as pulmonary emphysema or chronic bronchitis, with cytokeratin 18 protein to induce the formation of immune complex; and

(b) diagnosing said chronic obstructive pulmonary disease in the subject by detecting the above immune complex to determine the presence of autoantibodies to said cytokeratin 18 protein,

wherein the cytokeratin 18 protein has an amino acid sequence of SEQ ID NO: 2, wherein chronic obstructive pulmonary disease is diagnosed when the patients complain of typical clinical symptoms, including cough, sputum, and dyspnea, and their FEV1 measured following the inhalation of bronchodilator showed less than 80% of predictive value and the ratio of FEV1/FVC was less than 70% on pulmonary function test.

In step (a) of the present diagnosing method, the biological samples can include any fluid which can be isolated and collected from human bodies, such as blood, plasma, serum, urine, tears, salivar, sputum, nasal secretion, bronchial secretion, bronchial washing fluid, pulmonary secretion, alveolus washing fluid, etc.

The formation of immune complex can use immunoblotting, ELISA, etc., and can use any methods for detecting the antigen-antibody reaction, which are ordinary in the art.

Step (b) of the present diagnosing method is a step determining the formation of immune complex by inducing color reactions or detecting fluorescence of immune complex, and it can use any methods for detecting antigen-antibody reaction, which are ordinary in the art. If the presence of immune complex is confirmed, this result means that the subject has COPD. For the positive control result, instead of the subject's biological samples, what can be used are polyclonal antibodies or monoclonal antibodies to cytokeratin 18 protein wherein the cytokeratin 18 protein has an amino acid sequence of SEQ ID NO: 2 from mammals, or human s biological samples of which autoantibodies to cytokeratin 18 protein wherein the cytokeratin 18 protein has an amino acid sequence of SEQ ID NO: 2 are positive. For the negative control result, what can be used are antibodies or buffers other than the antibodies to cytokeratin 18 protein wherein the cytokeratin 18 protein has an amino acid sequence of SEQ ID NO: 2.

From the prior reports of immunological studies for other autoimmune dieases, it is known that in case of patients showing IgG autoantibodies to specific autoantigen proteins in blood, a considerable number of patients has antigen-specific T-cell reaction to the same autoantigen protein, and it is useful to test delayed type hypersensitivity after intradermal injection of the same autoantigen proteins reacting with IgG autoantibodies in the patients (Beales PE et al, Autoimmunity 2000;32:109-113). In prior arts, skin tests of tuberculin type have been used, in which tests were performed by injecting specific antigen to subjects, intradermally, and testing the injected skin part after 24 to 72 hours of the injection to measuring the size of skin swelling (induration), thus determining the presence of delayed-type hypersensitivity reaction or late-onset skin reaction to the above antigen.

Accordingly, the diagnostic composition comprising cytokeratin 18 protein wherein the cytokeratin 18 protein has an amino acid sequence of SEQ ID NO: 2 can be applied to the use of the diagnostic composition to detecting patients showing autoimmune response to cytokeratin 18 protein among patients with COPD by measuring the size of skin swelling after 24 to 72 hours of the intradermal injection of cytokeratin 18 protein.

Also, the specification provides a method of diagnosing COPD by using a diagnostic composition comprising cytokeratin 18 protein.

The described method of diagnosing chronic COPD comprises the following steps:

a) selecting a subject suspected to have COPD;
b) injecting intradermally the diagnostic compositions comprising cytokeratin 18 protein and
c) diagnosing the COPD by examining the injected skin part after 24 to 72 hours of the injection and detecting the presence of the skin swelling and measuring the size of skin swelling, thus determining the presence of delayed-type hypersensitivity reaction or late skin reaction to cytokeratin 18 protein.

By using the present diagnostic composition and diagnosing method, the result for the presence of COPD can be obtained easily and correctly determined. Therefore, it can be used effectively for clinical study in a large scale. Also, it can be used to screen therapeutic agents or to develope treatment methods for COPD.

The present invention provides the use of a composition for screening a therapeutic agent for COPD, comprising cytokeratin 18 protein wherein the cytokeratin 18 protein has an amino acid sequence of SEQ ID NO: 2.

The compositions for screening a therapeutic agent can comprise buffer or reaction solution which makes maintain physiological acivity or structure of proteins, besides cytokerat in 18 protein wherein the cytokeratin 18 protein has an amino acid sequence of SEQ ID NO: 2. Also, the composition can be provided in the form of powder, or in the solubilized state in an appropriate buffer, or maintained at 4°C, in order to maintain stability.

The present compositions for screening a therapeutic agent enable the selection of a therapeutic agent for COPD, by screening the material which inhibits binding between cytokeratin 18 protein wherein the cytokeratin 18 protein has an amino acid sequence of SEQ ID NO: 2 and autoantibodies to cytokeratin 18 wherein the cytokeratin 18 protein has an amino acid sequence of SEQ ID NO: 2 in the biological samples obtained from the patients with COPD, or inhibits cytotoxic reaction of the autoantibodies to cytokeratin 18 protein wherein the cytokeratin 18 protein has an amino acid sequence of SEQ ID NO: 2 expressing cells.

Also, the specification describes a method of screening a therapeutic agent for COPD, by using compositions comprising one or more of cytokeratin 18 protein, antibodies to cytokeratin 18 protein, or autoantibodies to cytokeratin 18 from the patients with COPD, as target materials.

The method of screening a therapeutic agent for COPD comprises the following step:

(a) isolating autoantibodies from biological samples obtained from the patients with COPD;
(b) contacting test materials with the autoantibodies obtained in step (a); and

(c) selecting a therapeutic agent for COPD by determining whether the test materials can inhibit binding of the autoantibodies to cytokeratin 18 protein, or inhibit cytotoxic effects of the autoantibodies to cytokeratin 18 protein-expressing cells.

In the described method of screening a therapeutic agent, the test materials can be nucleic acids, proteins, extracts, or compounds which are expected to have possibilities of inhibitor for COPD according to ordinary selecting method, or randomly selected.

In the described method for screening a therapeutic agent, for the confirmation of the reaction between the composition for screening and the test materials, common methods used to confirm protein-protein reaction including antigen-antibody reaction or protein-compound reaction can be used.

For example, the following methods can be used: a method which reacts cytokeratin 18 proteins or autoantibodies with the test material, thereby determining the activity, yeast two-hybrid method, screening method of phage-displayed peptide clone binding to cytokeratin 18 proteins, high throughput screening (HTS) method using natural product, chemical library etc., drug hit HTS method, cell-based screening method, or screening method using DNA array, etc.

In present inventon, matters relating to genetic engineering technology will be more clear from the contents of Sambrook' s literature etc. (Sambrook, et al., Molecular Cloning, A Laboratory Manual. Cold Spring Harbor laboratory Press, Cold Spring Harbor, N. Y. (2001)).

1. A pharmaceutical composition for use in a method for preventing or treating chronic obstructive pulmonary disease comprising cytokeratin 18 protein as an active ingredient, wherein the cytokeratin 18 protein has an amino acid sequence of SEQ ID NO: 2.

2. The composition for use according to item 1, wherein the chronic obstructive pulmonary disease is pulmonary emphysema or chronic bronchitis.

3. The use of cytokeratin 18 protein as an active ingredient for the manufacture of a pharmaceutical composition for use in a method for preventing or treating chronic obstructive pulmonary disease, wherein the cytokeratin 18 protein has an amino acid sequence of SEQ ID NO: 2.

4. The use of according to item 3, wherein the chronic obstructive pulmonary disease is pulmonary emphysema or chronic bronchitis.

5. An *in vitro* method of diagnosing chronic obstructive pulmonary disease by using diagnostic compositions containing cytokeratin 18 protein as an active ingredient, comprising the steps of:

(a) contacting a biological sample of a subject suspected to have chronic obstructive pulmonary disease, with cytokeratin 18 protein to induce the formation of immune complex; and

(b) diagnosing said chronic obstructive pulmonary disease in the subject by detecting the above immune complex to determine the presence of autoantibodies to said cytokeratin 18 protein,

wherein the cytokeratin 18 protein has an amino acid sequence of SEQ ID NO: 2, wherein chronic obstructive pulmonary disease is diagnosed when the patients complain of typical clinical symptoms, including cough, sputum, and dyspnea, and their FEV1 measured following the inhalation of bronchodilator showed less than 80% of predictive value and the ratio of FEV1/FVC was less than 70% on pulmonary function test.

6. The method of item 5, wherein the chronic obstructive pulmonary disease is pulmonary emphysema or chronic bronchitis.

7. A use of a composition comprising cytokeratin 18 as target material for screening a therapeutic agent for chronic obstructive pulmonary disease, wherein cytokeratin 18 protein has the amino acid sequence of SEQ ID NO: 2.

8. The use according to item 7, wherein the chronic obstructive pulmonary disease is pulmonary emphysema or chronic bronchitis.

9. An *in vitro* method to aid in the detection of chronic obstructive pulmonary disease in a human subject, comprising the steps of:

(a) contacting a biological sample from the human subject with the cytokeratin 18 protein to form an immune complex between the cytokeratin 18 protein and the autoantibodies biological sample, and

(b) determining the presence of the autoantibodies against said cytokeratin 18 protein in the biological sample by detecting the immune complex, wherein the presence of the autoantibodies bears a positive correlation with the existence of chronic obstructive pulmonary disease in the human subject,

wherein the cytokeratin 18 protein has an amino acid sequence of SEQ ID NO: 2, wherein chronic obstructive pulmonary disease is diagnosed when the patients complain of typical clinical symptoms, including cough, sputum, and dyspnea, and their FEV1 measured following the inhalation of bronchodilator showed less than 80% of predictive value and the ratio of FEV1/FVC was less than 70% on pulmonary function test.

10. The method of item 9, wherein the chronic obstructive pulmonary disease is pulmonary emphysema or chronic bronchitis.

Advantageous effects

As decribed in the above, cytokeratin 18 protein is identified as an autoantigen recognized by autoantibodies in the serum samples of patients having COPD. Thus, a pharmaceutical composition of the present invention cytokeratin 18 wherein the cytokeratin 18 protein has an amino acid sequence of SEQ ID NO: 2 protein as an active ingredient can be used as a medicament for preventing, alleviating, and treating COPD through the inhibition of autoantibody-induced cytotoxicity to airway epithelial cells and the inhibition of the secondary development of chronic airway epithelial inflammation induced by the formation of immune complexes consisted of autoantibodies and autoantigen.

Also, a diagnostic composition comprising cytokeratin 18 proteinwherein the cytokeratin 18 protein has an amino acid sequence of SEQ ID NO: 2 can be used for detecting, diagnosing or classifying COPD.

Description of Drawings

Fig. 1 shows immunoblot analysis of human airway epithelial cell (A549) proteins reacting with IgG autoantibodies in serum samples of healthy controls, pulmonary emphysema patients having normal pulmonary function, and patients with COPD;

Fig. 2 shows immunoblot detection of IgG autoantibodies to recombinant human cytokeratin 18 protein in serum samples of healthy controls, pulmonary emphysema patients with normal pulmonary function, and patients with COPD;

Fig. 3 shows the bindings of IgG autoantibodies in serum samples of COPD with the recombinant human cytokeratin 18 proteins and the commercially available purified bovine cytokeratin 18 protein detected by immunoblot anlaysis;

Fig. 4 shows the detection of antibody-induced cytotoxicity to human airway epithelial cells (A549) by IgG antibodies purified from serum samples of patients with COPD;

Fig. 5 shows inhibitions of IgG autoantibody-induced cytotoxicity to airway epithelial cells (A549) when the IgG antibodies from patients with COPD were adsorbed with human cytokeratin 18 protein prior to addition to airway epithelial cells;

Best Mode

Hereinafter, the present invention is more specifically explained by the Examples. However, the following examples are provided for the purpose of illustration and are not intended to limit the scope of the present invention.

[EXAMPLE 1] Detection of IgG autoantibodies to cytokeratin 18 protein in serum samples from patients with COPD

(1-1) Subjects

Subjects

The inventors examined serum samples obtained from 9 patients with chronic obstructive pulmonary disease (COPD), 2 patients with pulmonary emphysema showing normal pulmonary function and 58 healthy controls. Diagnosis of COPD was determined by recently reported criterion of NHLBI/WHO GOLD Workshop summary (Am J Respir Crit Care Med 2001;163:1256-1276). Both of 2 patients with pulmonary emphysema had smoking history of more than 10 pack years. They have been clinically complained dyspnea symptom and chest X-ray showed findings compatible with pulmonary emphysema. However, they had normal findings with values of FEV1 (forced expiratory volume in one second) and FVC (forced vital capacity) were greater than 80% of predictive value on pulmonary function test. All 9 patients with COPD had clinical histories compatible with COPD such as cough, sputum, dyspnea, etc. Also, their FEV1 measured following the inhalation of bronchodilator showed less than 80% of predictive value on pulmonary function test, and the ratio of FEV1/FVC was less than 70%. All patients with COPD had smoking history of more than 10 pack years, but had no signs of tuberculosis or other pulmonary diseases in chest X-ray. The serum samples of the patients with COPD were stored at -20 °C.

(1-2) Cell Culture

The human airway epithelial cell line A549 (ATCC CCL-185; Giard et al, J Natl Cancer Inst 1973; 51:1417-23) was obtained from American Type Culture Collection (ATTC; VA, USA) and was cultured as recommend by ATCC.

(1-3) Extraction of cell protains and immunoblot analysis

Immunoblot analysis was performed with human airway epithelial cells and the serum samples obtained from healthy controls and patients with COPD according to the above Example (1-1), to identify human airway epithelial cell proteins which bind to IgG autoantibodies in the serum.

Firstly, in order to extract proteins from the human airway epithelial cell A549 in the above (1-2), the cultured cells were lysed in lysis buffer containing 10 mM Tris/HCl, pH 7.2, 2% sodium dodecyl sulphate (SDS), 158 mM NaCl, and 10 mM dithiothreitol.

The proteins in cell lysates were separated by discontinuous Sodium Dodecyl Sulfate-PolyacrylAmide Gel Electrophoresis (SDS-PAGE). Following electrophoresis, proteins were transferred onto a polyvinylidine difluoride (PVDF) membrane (Bio-Rad Laboratories, Hercules, CA). After the transfer, the PVDF membrane was treated with Tris-buffered saline (TBS) containing 5% bovine serum, 10% powdered skim milk and 0.1% Tween 20 for one or more hours, to block non-specific bindings of proteins to PVDF, and then the PVDF membrane was cut in 4mm strips. The cut strips were reacted with patients' serum diluted in the same buffer in 1:100 (v/v) for 2 hours at room temperature. After washing, the membrane was incubated with alkaline phosphates-conjugated goat anti-human IgG antibodies (Sigma Chemical Co., St. Louis, MO) for 2 hours at room temperature. After the final washing, the membrane was stained with a BCIP/NBT solution (5-bromo-4-chloro-3-indoyl phosphate/nitro blue tetrazolium; Sigma) for 5 minutes to detect the proteins reacted with patients' serum. To compare with results from patients with other disease, a serum sample from a patient with nonallergic asthma that a detection of IgG autoantibodies to cytokeratin 18 has been confirmed by previous report of inventors (Nahm DH, et al. Am J Respir Crit Care Med 2002;165:1536-9) was also included in the experiments.

In order to minimize technical errors in detection rate of each test, each test comprised a positive standard serum and negative control serum. The results of the tests were assessed by at least two investigators independently with naked eyes. When test serum showed strong band to certain airway epithelial cell protein compared to negative control serum, the stained intensity of the band were the same as or stronger than that of positive standard serum, and the result read by the two investigators corresponded to each other, which case was defined as the positive detection of autoantibodies.

Mouse monoclonal antibody to cytokeratin 18 protein (clone CK5, Sigma Chemical Co., St. Louis, MO), goat antibody to human alpha-enolase protein (Santa Cruz Biotechnology, Santa Cruz, CA) and their alkaline phosphatase-conjugated secondary antibodies were included in each experiment to confirm the location of the reacting autoantigen proteins when the detection results of the autoantibodies to airway epithelial cell proteins were read.

The target autoantigen protein was determined by comparing and analyzing the results of immunoblot localization of airway epithelial cell proteins on the PVDF membrane reacting with IgG autoantibodies of patients and airway epithelial cell proteins reacting with mouse monoclonal antibody to cytokeratin 18 protein (clone CK5, Sigma Chemical Co., St. Louis, MO) or goat antibody to human alpha-enolase protein (Santa Cruz Biotechnology, Santa Cruz, CA) in the points of staining patterns and molecular weights.

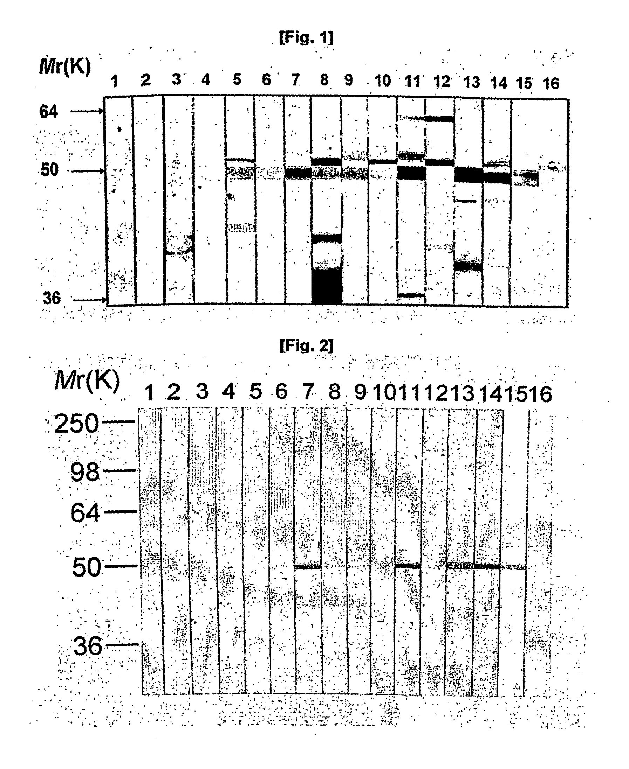

Fig. 1 shows result of immunoblot analysis of airway epithelial cell (A549) proteins reacting with IgG autoantibodies in serum samples of healthy controls, patients with pulmonary emphysema showing normal pulmonary function, and patients with COPD. Lanes 1 and 2 show the results for serum samples of healthy controls, lanes 3 and 4 show the results for serum samples of patients with pulmonary emphysema showing normal pulmonary function, lanes 5-13 show the results for serum samples of patients with COPD, and lane 14 shows the result for a serum sample of a patient with bronchial asthma, lane 15 shows the result for mouse monoclonal antibodies to cytokeratin 18 protein, and lane 16 shows the result for goat antibodies to alpha-enolase.

Fig. 1 shows that IgG autoantibodies to protein band corresponding to cytokeratin 18 protein identified by mouse monoclonal antibodies were detected in 8 (89%) of total 9 patients with chronic obstructive pulmonary disease. This result showed significantly higher detection rate than that of healthy controls (5 of 58 healthy control; 8.6%) (chi-square test, p<0.05).

(1-4) Detection of autoantibodies in the blood samples of patients with COPD using recombinant human cytokeratin 18 protein

By using the above subjects' serum of (1-3) and commercially available human recombinant cytokeratin 18 protein (Research Diagnostics INC., Pleasant Hill Road Flanders, NJ) produced from *E. coli* by the genetic engineering technology, immunoblot was performed in same sample arrangement as in Fig 1. The results are shown in Fig. 2.

Fig. 2 shows immunoblot detection of IgG autoantibodies to recombinant human cytokeratin 18 protein using serum samples of healthy controls, patients with pulmonary emphysema showing normal pulmonary function, and patients with COPD. Lanes 1 and 2 show the results for serum samples of healthy controls, lanes 3 and 4 show the results for serum samples of patients with pulmonary emphysema showing normal pulmonary function, lanes 5-13 show the results for serum samples of patients with COPD, and lane 14 shows the result for a serum sample of a patient with bronchial asthma, lane 15 shows the result for mouse monoclonal antibodies to cytokeratin 18 protein, and lane 16 shows the result for goat antibodies to alpha-enolase.

As shown in Fig. 2, IgG autoantibodies to recombinant human cytokeratin 18 protein were clearly detected in 3 (33.3%) of 9 patients with COPD (lanes 7,11, and 13). , IgG autoantibodies to recombinant human cytokeratin 18 protein were also detected in other 5 patients with COPD (lanes 5,6,8,9,10 in Fig. 2) and two patients with pulmonary emphysema showing normal pulmonary function (lane 3,4 in Fig. 2).

As the results of Fig. 2 were consistent with the results of Fig. 1, it was confirmed that the autoantigen reacting with IgG autoantibodies in serum samples from patients with COPD was human cytokeratin 18 protein.

As shown in Fig. 1 and Fig. 2, IgG autoantibodies to recombinant cytokeratin 18 protein or cytokeratin 18 protein in airway epithelial cells were positively detected in patients with COPD.

Thus, it was recomfirmed that the autoantigen reacting with IgG autoantibodies in the serum samples of patients with COPD was cytokeratin 18 protein (Fig. 2).

[Example 2] Immunoblot analysis for bovine cytokeratin 18 protein

In order to determine whether IgG autoantibodies in serum samples of patients with COPD are selectively reacted with human cytokeratin 18 protein or they could react with cytokeratin 18 proteins from other mammal, the inventors performed immunoblot analysis.

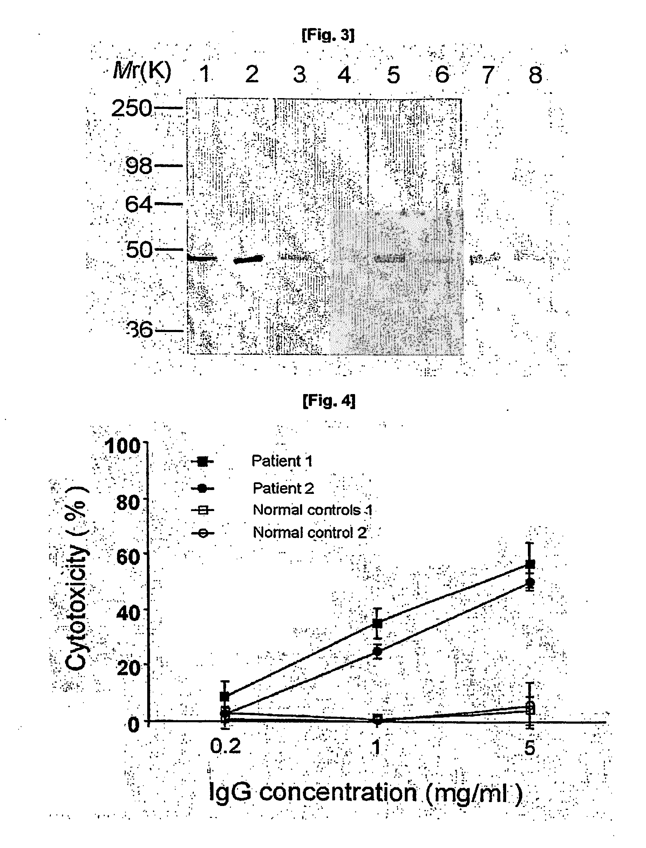

Reaction of IgG autoantibodies in serum samples from 3 patients with COPD with commercially available recombinant human cytokeratin 18 protein and purified bovine cytokeratin 18 protein (Research Diagnostics INC., Pleasant Hill Road Flanders, NJ) was detected and compared by immunoblot analysis, according to the method of (1-3). The results are shown in Fig. 3.

Fig. 3 shows immunoblot results that show comparing the reaction of IgG autoantibodies in serum samples of patients with COPD with recombinant human cytokeratin 18 protein and purified bovine cytokeratin 18 protein. Lanes 1, 3, 5 and 7 show the results of the recombinant human cytokeratin 18 protein, and lanes 2, 4, 6, and 8 show the results of purified bovine cytokeratin 18 protein. Lanes 1 & 2, lanes 3 & 4, and lanes 5 & 6 show the results obtained by reacting with 3 serum samples of 3 patients with COPD, and lanes 7 & 8 show the result of reacting with mouse monoclonal antibodies to cytokeratin 18 protein.

From the results obtained by using the serum samples of 3 patients with COPD, it was confirmed that IgG autoantibodies to cytokeratin 18 in their serum samples could react with bovine cytokeratin 18 protein having the same molarcular weight of human cytokeratin 18 protein.

As shown in the above, the test results of autoantigen-autoantibodies using human recombinant human cytokeratin 18 protein and purified bovine cytokeratin 18 protein corresponded to each other. Thus, it was confirmed that bovine cytokeratin 18 protein could be used to detect autoantibodie in serum samples of patients with COPD.

[Example 3] Inhibition of IgG antibody-induced cytotoxicity to airway epithelial cells by the administration of cytokeratin 18 protein

(3-1) Confirmation of cytotoxicity to airway epithelial cells by IgG autoantibodies

Cytotoxicity to airway epithelial cell by IgG autoantibodies was measured by the microcytotoxicity method using Terasaki tray and modifying prior reported method (Martin S, et al; Tissue Antigens 1991;37:152-155).

In the above test, IgG antibodies were isolated from plasma samples of 2 patients with COPD, and a healthy control, where patients with COPD had IgG autoantibodies to human cytokeratin 18 protein.

IgG antibodies of 1 healthy control were isolated to have a purity of 90%, by using ethanol precipitation and ultrafiltration according to prior preparing method of IgG antibodies (Lebing W et al, Vox Sang 2003;84:193-201). IgG antibodies of 2 patients with COPD were isolated in purity of greater than 95% by affinity chromatography using Protein A column. The purity of the isolated IgG antibodies was determined by SDS-PAGE and protein staining. Also, the amount of endotoxin in the isolated IgG antibody solution was measured quantitatively by using LAL (Limulus amebocyte lysate). As a result, the contamination of endotoxin was not detected.

Human IgG antibodies used for intravenous administration (IVglobulin, Green Cross Pharmaceutical Co., Korea) were commercially obtained and used as control IgG antibodies, where the control IgG antibodies were isolated from plasma of a large mumber of blood donors, to have a purity of more than 95%.

First of all, the above human IgG antibodies were diluted by using DMEM/F12 medium at concentrations of 5mg/mℓ, 1mg/ℓ, and 0.2mg/mℓ. Then, the diluted samples were put into 96-well Terasaki tray at a ratio of 1 μℓ/well in quadruplicate. Also, samples containing only DMEM/F12 medium were put in 96-well Terasaki tray in quadruplicate as negative controls.

The cultured human airway epithelial cell line A549 was treated with Trypsin/EDTA and isolated, then 1 μℓ (2000 cells/μl) of the solution was put into each of above wells. In order to prevent evaporation of the solution, 5 μℓ of mineral oil was put into the wells. And then, the wells were reacted for 2 hours and 30 minutes. 5% eosin Y dye (Sigma Chemical Co.) was put into the wells at a ratio of 5 μℓ/well to stain the cells. Then, formalin solution was put into the wells at a ratio of 5 μℓ/well to fix the stained cells. A coverslide was covered and the number of cells without damage of cell membrane was counted by light microscope to determine cytotoxicity.

The cytotoxicity to airway epithelial cell by IgG antibodies was expressed as cytolysis % according to following equation, by comparing mean number of cells in wells reacting with the samples and mean number of cells in negative control wells containing only DMEM/F12 medium.

【Equation 1】

Cytotoxicity (cytolysis %) = [(mean number of cells in negative control wells - mean number of cells in wells containg samples) / mean number of cells in negative control wells] x 100

The result of the test was obtained in quadruplicate to each sample, and then expressed in terms of mean and standard deviation.

Fig. 4 show that IgG antibodies isolated from plasma samples of 2 patients with COPD (patient 1 and patient 2) show significantly higher cytotoxicity than commercial IgG antibodies obtained from multiple healthy donors used for intravenous administration (IVglobulin; normal controls 1) or IgG antibodies isolated from plasma of a healthy control (normal control 2) under the conditions of 1μg/well and 5μg/well (t-test, p < 0.05).

(3-2) Inhibition of IgG autoantibody-induced cytotoxicity to airway epithelial cell by cytokeratin 18 protein

In order to absorb autoantibodies to cytokeratin 18 protein from patients with COPD, 50 μg of the human cytokeratin 18 protein purified according to the previous report of the inventors (Nahm DH, et al. Am J Respir Crit Care Med 2002;165:1536-9) and 50 μg of the purified human cytokeratin 19 protein as a negative control were subjected to electrophoresis and transferred to PVDF membrane. PVDF membranes containing human cytokeratin 18 protein or human cytokeratin 19 protein were cutted into small pieces and transferred to eppendorf tubes separately.

50 μg of IgG antibodies which were isolated from serum samples of 2 patients with COPD and diluted in DMEM/F12 medium to have a concentration of 5 mg/mℓ were mixed with PVDF membrane pieces containing either 50 μg of human cytokeratin 18 protein or human cytokeratin 19 protein, and then, the mixtures were reacted for 16 hours at 4°C. After the reaction, supernatant was collected by centrifugation, and the cytotoxicity to human airway epithelial cell by IgG antibodies was measured in the above method (3-1).

The result of the experiment was obtained in 6 independent experiments of each samples, and then, expressed in terms of mean and standard deviation.

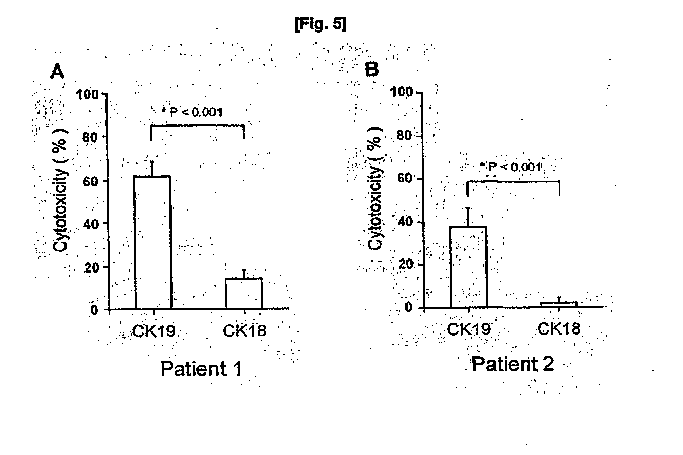

Fig. 5 shows that when the IgG antibodies of patients with COPD (patient 1, 2) were adsorbed with human cytokeratin 18 protein prior to the addition to airway epithelial cells, the cytotoxicity to airway epithelial cell by IgG autoantibodies (shown as "CK18" in each graph) was significantly reduced compared to the case of adsorbtion with the same amount of human cytokeratin 19 protein used as a control antigen (shown as "CK19" in each graph) (Table 1) (t-test, p < 0.001).

[TABLE 1]

| Patients | Absorption with cytokeratin 19 protein (cytolysis %) | Absorption with cytokeratin 18 protein (cytolysis %) |
|---|---|---|
| Patient 1 | 61.9 ± 6.4* | 13.7 ± 4.5 |
| Patient 2 | 37.2 ± 6.2* | 1.8 ± 2.7 |
| *: P<0.01 | | |

Above results show that cytokeratin 18 protein can be used for the inhibition of cytotoxic reaction to airway epithelial cell induced by IgG autoantibodies to cytokeratin 18 protein in the blood of patients with COPD.

Accordingly, it is evident that the cytotoxic effect and the inflammatory effect of IgG autoantibodies can be repressed if the cytokeratin 18 protein wherein the cytokeratin 18 protein has an amino acid sequence of SEQ ID NO: 2 of the present invention is administered into the patients with COPD.

Industrial Applicability

As decribed in the above, cytokeratin 18 protein is identified as an autoantigen recognized by autoantibodies in the serum samples of patients having COPD. Thus, a pharmaceutical composition of the present invention cytokeratin 18 wherein the cytokeratin 18 protein has an amino acid sequence of SEQ ID NO: 2 protein as an active ingredient can be used as a medicament for preventing, alleviating, and treating COPD through the inhibition of autoantibody-induced cytotoxicity to airway epithelial cells and the inhibition of the secondary development of chronic airway epithelial inflammation induced by the formation of immune complexes consisted of autoantibodies and autoantigen.

Also, the present invention can be used for a pharmaceutical formulation comprising cytokeratin 18 protein wherein the cytokeratin 18 protein has an amino acid sequence of SEQ ID NO: 2 or fragments thereof to protect patients with COPD. The present invention also can be used to identify a pharmaceutical compound capable of inhibiting the binding ability of autoantibodies to cytokeratin 18 from patients with COPD to cytokeratin 18 protein wherein the cytokeratin 18 protein has an amino acid sequence of SEQ ID NO: 2 or cytokeratin 18-expressing cells wherein the cytokeratin 18 protein has an amino acid sequence of SEQ ID NO: 2.

## Claims

1. A pharmaceutical composition for use in a method for preventing or treating chronic obstructive pulmonary disease comprising cytokeratin 18 protein as an active ingredient, wherein the cytokeratin 18 protein has an amino acid sequence of SEQ ID NO: 2.

2. The composition for use according to claim 1, wherein the chronic obstructive pulmonary disease is pulmonary emphysema or chronic bronchitis.

3. The use of cytokeratin 18 protein as an active ingredient for the manufacture of a pharmaceutical composition for use in a method for preventing or treating chronic obstructive pulmonary disease, wherein the cytokeratin 18 protein has an amino acid sequence of SEQ ID NO: 2.

4. The use of according to claim 3, wherein the chronic obstructive pulmonary disease is pulmonary emphysema or chronic bronchitis.

5. An *in vitro* method of diagnosing chronic obstructive pulmonary disease by using diagnostic compositions containing cytokeratin 18 protein as an active ingredient, comprising the steps of:

   (a) contacting a biological sample of a subject suspected to have chronic obstructive pulmonary disease, with cytokeratin 18 protein to induce the formation of immune complex; and
   (b) diagnosing said chronic obstructive pulmonary disease in the subject by detecting the above immune complex to determine the presence of autoantibodies to said cytokeratin 18 protein,

   wherein the cytokeratin 18 protein has an amino acid sequence of SEQ ID NO: 2, wherein chronic obstructive pulmonary disease is diagnosed when the patients complain of typical clinical symptoms, including cough, sputum, and dyspnea, and their FEV1 measured following the inhalation of bronchodilator showed less than 80% of predictive value and the ratio of FEV1/FVC was less than 70% on pulmonary function test.

6. The method of claim 5, wherein the chronic obstructive pulmonary disease is pulmonary emphysema or chronic bronchitis.

7. A use of a composition comprising cytokeratin 18 as target material for screening a therapeutic agent for chronic obstructive pulmonary disease, wherein cytokeratin 18 protein has the amino acid sequence of SEQ ID NO: 2.

8. The use according to claim 7, wherein the chronic obstructive pulmonary disease is pulmonary emphysema or chronic bronchitis.

9. An *in vitro* method to aid in the detection of chronic obstructive pulmonary disease in a human subject, comprising the steps of:

   (a) contacting a biological sample from the human subject with the cytokeratin 18 protein to form an immune complex between the cytokeratin 18 protein and the autoantibodies biological sample, and
   (b) determining the presence of the autoantibodies against said cytokeratin 18 protein in the biological sample by detecting the immune complex, wherein the presence of the autoantibodies bears a positive correlation with the existence of chronic obstructive pulmonary disease in the human subject,

   wherein the cytokeratin 18 protein has an amino acid sequence of SEQ ID NO: 2, wherein chronic obstructive pulmonary disease is diagnosed when the patients complain of typical clinical symptoms, including cough, sputum, and dyspnea, and their FEV1 measured following the inhalation of bronchodilator showed less than 80% of predictive value and the ratio of FEV1/FVC was less than 70% on pulmonary function test.

10. The method of claim 9, wherein the chronic obstructive pulmonary disease is pulmonary emphysema or chronic bronchitis.

**Patentansprüche**

1. Arzneimittel zur Verwendung in einem Verfahren für die Prävention oder Behandlung einer chronisch obstruktiven Lungenerkrankung, umfassend Cytokeratin-18-Protein als Wirkstoff, wobei das Cytokeratin-18-Protein eine Aminosäuresequenz der SEQ ID NO:2 aufweist.

2. Zusammensetzung zur Verwendung nach Anspruch 1, wobei die chronisch obstruktive Lungenerkrankung Lungenemphysem oder chronische Bronchitis ist.

3. Verwendung von Cytokeratin-18-Protein als Wirkstoff für die Herstellung eines Arzneimittels zur Verwendung in einem Verfahren für die Prävention oder Behandlung einer chronisch obstruktiven Lungenerkrankung, wobei das Cytokeratin-18-Protein eine Aminosäuresequenz der SEQ ID NO:2 aufweist.

4. Verwendung nach Anspruch 3, wobei die chronisch obstruktive Lungenerkrankung Lungenemphysem oder chronische Bronchitis ist.

5. *In vitro*-Verfahren für die Diagnose einer chronisch obstruktiven Lungenerkrankung unter Verwendung diagnostischer Zusammensetzungen, die Cytokeratin-18-Protein als Wirkstoff enthalten, umfassend die Schritte:

   (a) Inkontaktbringen einer biologischen Probe eines Individuums, bei dem der Verdacht besteht, dass es an einer chronisch obstruktiven Lungenerkrankung leidet, mit Cytokeratin-18-Protein, um die Bildung eines Immunkomplexes zu induzieren; und
   (b) Diagnostizieren der chronisch obstruktiven Lungenerkrankung bei dem Individuum durch Nachweisen des vorstehenden Immunkomplexes, um das Vorhandensein von Autoantikörpern gegen das Cytokeratin-18-Protein zu bestimmen,

   wobei das Cytokeratin-18-Protein eine Aminosäuresequenz der SEQ ID NO:2 aufweist, wobei die chronisch obstruktive Lungenerkrankung diagnostiziert wird, wenn die Patienten über typische klinische Symptome, einschließlich Husten, Sputum und Dyspnoe, klagen und ihr im Anschluss an die Inhalation eines Bronchodilatators gemessener FEV1-Wert weniger als 80% des vorhergesagten Wertes zeigte und das FEV1/FVC-Verhältnis beim Lungenfunktionstest niedriger als 70% war.

**6.** Verfahren nach Anspruch 5, wobei die chronisch obstruktive Lungenerkrankung Lungenemphysem oder chronische Bronchitis ist.

**7.** Verwendung einer Zusammensetzung, die Cytokeratin-18 als Zielsubstanz für das Screenen eines therapeutischen Mittels gegen eine chronisch obstruktive Lungenerkrankung umfasst, wobei Cytokeratin-18-Protein die Aminosäuresequenz der SEQ ID NO:2 aufweist.

**8.** Verwendung nach Anspruch 7, wobei die chronisch obstruktive Lungenerkrankung Lungenemphysem oder chronische Bronchitis ist.

**9.** *In vitro*-Verfahren zur Unterstützung des Nachweises einer chronisch obstruktiven Lungenerkrankung bei einem menschlichen Individuum, umfassend die Schritte:

> (a) Inkontaktbringen einer biologischen Probe des menschlichen Individuums mit dem Cytokeratin-18-Protein zur Bildung eines Immunkomplexes aus dem Cytokeratin-18-Protein und den Autoantikörpern der biologischen Probe, und
> (b) Bestimmen des Vorhandenseins der Autoantikörper gegen das Cytokeratin-18-Proteins in der biologischen Probe durch Nachweisen des Immunkomplexes, wobei das Vorhandensein der Autoantikörper in einer positiven Korrelation zum Vorliegen einer chronisch obstruktiven Lungenerkrankung bei dem menschlichen Individuum steht,

> wobei das Cytokeratin-18-Protein eine Aminosäuresequenz der SEQ ID NO:2 hat, wobei die chronisch obstruktive Lungenerkrankung diagnostiziert wird, wenn die Patienten über typische klinische Symptome, einschließlich Husten, Sputum und Dyspnoe, klagen und ihr im Anschluss an die Inhalation eines Bronchodilatators gemessener FEV1-Wert weniger als 80% des vorhergesagten Wertes zeigte und das FEV1/FVC-Verhältnis beim Lungenfunktionstest niedriger als 70% war.

**10.** Verfahren nach Anspruch 9, wobei die chronisch obstruktive Lungenerkrankung Lungenemphysem oder chronische Bronchitis ist.

**Revendications**

**1.** Composition pharmaceutique pour utilisation dans un procédé de prévention ou de traitement d'une bronchopneumopathie chronique obstructive comprenant la protéine cytokératine 18 en tant que principe actif, dans laquelle la protéine cytokératine 18 a une séquence d'acides aminés de SEQ ID NO:2.

**2.** Composition pour utilisation selon la revendication 1, dans laquelle la bronchopneumopathie chronique obstructive est un emphysème pulmonaire ou une bronchite chronique.

**3.** Utilisation de la protéine cytokératine 18 en tant que principe actif pour la fabrication d'une composition pharmaceutique pour utilisation dans un procédé de prévention ou de traitement d'une bronchopneumopathie chronique obstructive, dans laquelle la protéine cytokératine 18 a une séquence d'acides aminés de SEQ ID NO:2.

**4.** Utilisation selon la revendication 3, dans laquelle la bronchopneumopathie chronique obstructive est un emphysème pulmonaire ou une bronchite chronique.

**5.** Procédé *in vitro* de diagnostic d'une bronchopneumopathie obstructive chronique en utilisant des compositions diagnostiques contenant la protéine cytokératine 18 en tant que principe actif, comprenant les étapes suivantes .

> (a) la mise en contact d'un échantillon biologique d'un sujet suspecté de souffrir d'une bronchopneumopathie chronique obstructive avec la protéine cytokératine 18 pour induire la formation d'un complexe immun ; et
> (b) le diagnostic de ladite bronchopneumopathie chronique obstructive chez le sujet par la détection du complexe immun ci-dessus pour déterminer la présence d'autoanticorps dirigés contre ladite protéine cytokératine 18,

dans lequel la protéine cytokératine 18 a une séquence d'acides aminés de SEQ ID NO:2, dans lequel la bronchopneumopathie chronique obstructive est diagnostiquée lorsque les patients se plaignent de symptômes cliniques typiques, y compris une toux, des crachats et une dyspnée et que leur FEV1 mesuré après l'inhalation d'un bronchodilatateur a affiché moins de 80 % de la valeur prédictive et que le rapport FEV1/FVC a été inférieur à 70 % sur le test de la fonction pulmonaire.

6. Procédé selon la revendication 5, dans lequel la bronchopneumopathie chronique obstructive est un emphysème pulmonaire ou une bronchite chronique.

7. Utilisation d'une composition comprenant la cytokératine 18 comme matériau cible pour un criblage à la recherche d'un agent thérapeutique pour une bronchopneumopathie chronique obstructive, dans laquelle la protéine cytokératine 18 a la séquence d'acides aminés de SEQ ID NO:2.

8. Utilisation selon la revendication 7, dans laquelle la bronchopneumopathie chronique obstructive est un emphysème pulmonaire ou une bronchite chronique.

9. Procédé *in vitro* pour aider à la détection d'une bronchopneumopathie chronique obstructive chez un sujet humain, comprenant les étapes suivantes :

(a) la mise en contact d'un échantillon biologique provenant du sujet humain avec la protéine cytokératine 18 pour former un complexe immun entre la protéine cytokératine 18 et les auto-anticorps de l'échantillon biologique, et
(b) la détermination de la présence des auto-anticorps dirigés contre ladite protéine cytokératine 18 dans l'échantillon biologique par la détection du complexe immun, où la présence des auto-anticorps supporte une corrélation positive avec l'existence d'une bronchopneumopathie chronique obstructive chez le sujet humain,

dans lequel la protéine cytokératine 18 a une séquence d'acides aminés de SEQ ID NO:2, dans lequel la bronchopneumopathie chronique obstructive est diagnostiquée lorsque les patients se plaignent de symptômes cliniques typiques, y compris une toux, des crachats et une dyspnée et que leur FEV1 mesuré après l'inhalation d'un bronchodilatateur a affiché moins de 80 % de la valeur prédictive et que le rapport FEV1/FVC a été inférieur à 70 % sur le test de la fonction pulmonaire.

10. Procédé selon la revendication 9, dans lequel la bronchopneumopathie chronique obstructive est un emphysème pulmonaire ou une bronchite chronique.

[Fig. 1]

[Fig. 2]

[Fig. 3]

[Fig. 4]

[Fig. 5]

A

B

Patient 1

Patient 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03098211 A
- WO 2006073254 A
- WO 03023002 A

### Non-patent literature cited in the description

- *Am J Respir Crit Care Med,* 2001, vol. 163, 1256-1276
- *GOLD workshop summary, Am J Respir Crit Care Med 2001,* 2001, vol. 163, 1256-1276
- **0'BYRNE PM ; POSTMA DS.** *Am J Respir Crit Care Med,* 1999, vol. 159, S41-S66
- **HODGE S et al.** *Eur Respir J,* 2005, vol. 25, 447-54
- **WAGNER V et al.** *Acta Allergol,* 1965, vol. 20, 1-9
- **AGUSTI A et al.** *Thorax,* 2003, vol. 58, 832-834
- **TARASEVICIENE-STEWART L et al.** *Am J Respir Crit Care Med.,* 2005, vol. 171, 734-42
- **MOLL, R. et al.** *Cell,* 1982, vol. 31, 11-24
- **MOLL, R. et al.** *Cell,* 1992, vol. 31, 11-24
- **SAARLOOS, M.N. et al.** *Curr Eye Res,* 1999, vol. 19, 439-449
- **RYU ; SCANLON.** *Mayo Clin. Proc.,* 2001, vol. 76, 1144-1153
- **JEFFERY.** *Proc Am Thorac Soc,* 2004, vol. 1, 176-183
- Remington's Pharmaceutical Science. Mack Publishing Company
- **BEALES PE et al.** *Autoimmunity,* 2000, vol. 32, 109-113
- **SAMBROOK et al.** Molecular Cloning, A Laboratory Manual. Cold Spring Harbor laboratory Press, 2001
- **GIARD et al.** *J Natl Cancer Inst,* 1973, vol. 51, 1417-23
- **NAHM DH et al.** *Am J Respir Crit Care Med,* 2002, vol. 165, 1536-9
- **MARTIN S et al.** *Tissue Antigens,* 1991, vol. 37, 152-155
- **LEBING W et al.** *Vox Sang,* 2003, vol. 84, 193-201

Sequence Listing

<110> JEON, Sook-Yeong NAM, Dong-Ho

<120> Composition for prevention, treatment and diagnosis of chronic obstructive pulmonary disease

<130> NP06-0017

<160> 2

<170> KopatentIn 1.71

<210> 1
<211> 430
<212> PRT
<213> Homo sapiens

<400> 1

```
Met Ser Phe Thr Thr Arg Ser Thr Phe Ser Thr Asn Tyr Arg Ser Leu
 1               5               10              15

Gly Ser Val Gln Ala Pro Ser Tyr Gly Ala Arg Pro Val Ser Ser Ala
        20              25              30

Ala Ser Val Tyr Ala Gly Ala Gly Gly Ser Gly Ser Arg Ile Ser Val
        35              40              45

Ser Arg Ser Thr Ser Phe Arg Gly Gly Met Gly Ser Gly Gly Leu Ala
        50              55              60

Thr Gly Ile Ala Gly Gly Leu Ala Gly Met Gly Gly Ile Gln Asn Glu
 65              70              75              80

Lys Glu Thr Met Gln Ser Leu Asn Asp Arg Leu Ala Ser Tyr Leu Asp
                85              90              95

Arg Val Arg Ser Leu Glu Thr Glu Asn Arg Arg Leu Glu Ser Lys Ile
            100             105             110

Arg Glu His Leu Glu Lys Lys Gly Pro Gln Val Arg Asp Trp Ser His
```

115        120        125

Tyr Phe Lys Ile Ile Glu Asp Leu Arg Ala Gln Ile Phe Ala Asn Thr
   130        135        140

Val Asp Asn Ala Arg Ile Val Leu Gln Ile Asp Asn Ala Arg Leu Ala
145        150        155        160

Ala Asp Asp Phe Arg Val Lys Tyr Glu Thr Glu Leu Ala Met Arg Gln
      165        170        175

Ser Val Glu Asn Asp Ile His Gly Leu Arg Lys Val Ile Asp Asp Thr
      180        185        190

Asn Ile Thr Arg Leu Gln Leu Glu Thr Glu Ile Glu Ala Leu Lys Glu
      195        200        205

Glu Leu Leu Phe Met Lys Lys Asn His Glu Glu Glu Val Lys Gly Leu
   210        215        220

Gln Ala Gln Ile Ala Ser Ser Gly Leu Thr Val Glu Val Asp Ala Pro
225        230        235        240

Lys Ser Gln Asp Leu Ala Lys Ile Met Ala Asp Ile Arg Ala Gln Tyr
      245        250        255

Asp Glu Leu Ala Arg Lys Asn Arg Glu Glu Leu Asp Lys Tyr Trp Ser
      260        265        270

Gln Gln Ile Glu Glu Ser Thr Thr Val Val Thr Thr Gln Ser Ala Glu
      275        280        285

Val Gly Ala Ala Glu Thr Thr Leu Thr Glu Leu Arg Arg Thr Val Gln
   290        295        300

Ser Leu Glu Ile Asp Leu Asp Ser Met Arg Asn Leu Lys Ala Ser Leu
305        310        315        320

Glu Asn Ser Leu Arg Glu Val Glu Ala Arg Tyr Ala Leu Gln Met Glu
      325        330        335

```
Gln Leu Asn Gly Ile Leu Leu His Leu Glu Ser Glu Leu Ala Gln Thr
              340             345             350

Arg Ala Glu Gly Gln Arg Gln Ala Gln Glu Tyr Glu Ala Leu Leu Asn
              355             360             365

Ile Lys Val Lys Leu Glu Ala Glu Ile Ala Thr Tyr Arg Arg Leu Leu
              370             375             380

Glu Asp Gly Glu Asp Phe Asn Leu Gly Asp Ala Leu Asp Ser Ser Asn
385             390             395             400

Ser Met Gln Thr Ile Gln Lys Thr Thr Arg Arg Ile Val Asp Gly
              405             410             415

Lys Val Val Ser Glu Thr Asn Asp Thr Lys Val Leu Arg His
              420             425             430
```

<210> 2
<211> 429
<212> PRT
<213> Bos taurus

<400> 2

```
Met Ser Phe Ser Thr Gln Ser Thr Phe Ser Asn Tyr Arg Ser Leu Gly
1               5               10              15

Ser Val Gln Ser Ser Gly His Arg Val Arg Pro Val Ser Ser Ala Ala
              20              25              30

Ser Val Tyr Ala Gly Ala Gly Gly Ser Gly Ser Arg Ile Ser Val Ser
              35              40              45

Arg Thr Thr Ser Val Arg Gly Gly Trp Gly Ser Gly Asn Leu Gly Ala
              50              55              60

Gly Met Ala Gly Gly Leu Val Gly Val Gly Gly Ile Gln Gly Glu Lys
65              70              75              80
```

Glu Thr Met Gln Asp Leu Asn Asp Arg Leu Ala Ser Tyr Leu Glu Lys
                85                  90                  95

Val Arg Ser Leu Glu Ala Asp Asn Arg Arg Leu Glu Ser Lys Ile Arg
                100                 105                 110

Glu His Leu Glu Lys Lys Gly Pro Gln Val Arg Asp Trp Ala His Tyr
                115                 120                 125

Leu Lys Ile Ile Glu Asp Leu Arg Ala Gln Ile Phe Ala Asn Ser Val
                130                 135                 140

Asp Asn Ala Arg Ile Val Leu Gln Ile Asp Asn Ala Arg Leu Ala Ala
145                 150                 155                 160

Asp Asp Phe Arg Val Lys Tyr Glu Thr Glu Leu Ala Met Arg Gln Ser
                165                 170                 175

Val Glu Ser Asp Ile His Gly Leu Arg Lys Val Ile Asp Asp Thr Asn
                180                 185                 190

Val Thr Arg Leu Gln Leu Glu Thr Glu Ile Glu Ala Leu Lys Glu Glu
                195                 200                 205

Leu Leu Phe Met Lys Lys Asn His Glu Glu Glu Val Lys Gly Leu Gln
                210                 215                 220

Asn Gln Ile Ala Asn Ser Gly Leu Thr Val Glu Leu Asp Ala Pro Lys
225                 230                 235                 240

Pro Gln Asp Leu Ser Lys Ile Met Ala Asp Ile Arg Ala Gln Tyr Asp
                245                 250                 255

Glu Leu Ala Gln Lys Asn Arg Glu Glu Leu Asp Lys Tyr Trp Ser Gln
                260                 265                 270

Gln Ile Glu Glu Ser Thr Thr Val Val Thr Ser Gln Thr Ala Glu Ile
                275                 280                 285

```
Gly Ala Ala Glu Met Thr Leu Thr Glu Leu Arg Arg Thr Val Gln Ser
    290                 295             300

Leu Glu Ile Asp Leu Asp Ser Met Arg Asn Leu Lys Ala Ser Leu Glu
305             310             315                 320

Asn Ser Leu Arg Glu Val Glu Ala Arg Tyr Ala Met Gln Met Glu Gln
            325             330                 335

Leu Asn Gly Val Leu Leu His Leu Glu Ser Glu Leu Ala Gln Thr Arg
            340             345                 350

Ala Glu Gly Gln Arg Gln Thr Gln Glu Tyr Glu Ala Leu Leu Asn Val
            355             360                 365

Lys Val Lys Leu Glu Ala Glu Ile Asn Thr Tyr Arg Arg Leu Leu Glu
            370             375             380

Asp Gly Glu Asp Phe Ser Leu Gly Asp Ala Leu Asp Ser Ser Asn Ser
385             390             395                 400

Arg Gln Thr Ile Gln Lys Thr Thr Thr Leu Arg Leu Val Asp Gly Lys
                405             410                 415

Val Val Ser Glu Thr Ser Asp Thr Lys Val Leu Arg His
            420             425
```